# EUROPEAN PATENT APPLICATION

(11) **EP 0 848 999 A2**
(43) Date of publication of application: **24.06.1998**
(21) Application number: 97830670.2
(22) Date of filing: 12.12.1997
(51) Int. Cl.: B05B 9/04

(54) **Device for the atomization of liquid substances**

(30) Priority: 17.12.1996 IT PR960052
(71) Applicant: Schia, Lorenzo, 43048 S. Andrea Bagni (PR) (IT)
(72) Inventor: Schia, Lorenzo, 43048 S. Andrea Bagni (PR) (IT)
(74) Representative: Gotra, Stefano

(57) **Abstract**

The invention falls within the sector of devices for the atomization of liquid products, in particular for the disinfecting, deodorization, humidification and disinfestation of fixed or mobile environments or open areas.

The device comprises a submergible pump (9) contained in a tank (1) from which it draws the liquid to be conveyed to a plurality of atomization nozzles (12). The submergible pump (9) is powered by a solar panel (5) or by a battery (7) which is charged by the solar panel itself.

## Description

The present invention relates to a device for the atomization of liquid substances.

The main application of invention consists in the disinfecting, deodorization and humidification of closed premises, but the invention may also be used for the disinfestation of open areas, such as in the case where, for example, mosquitoes or other plant parasite animals must be eliminated.

One advantageous application consists in the deodorization of rubbish tips or areas where purification plants or, in general, treatment plants are in operation and produce odours or cause air pollution.

The device, in view of its characteristic feature that it may be powered autonomously may also be advantageously used on mobile means, for example in order to deodorize or disinfect railway carriages.

Atomization devices are already known, having a tank containing a liquid which is drawn by a pump operated by an electric motor located on a frame which supports it together with a suitable electric console. The electric motor is supplied with an alternating mains voltage, i.e. of 220 volts. The pump conveys the liquid under pressure to a plurality of atomization nozzles.

The devices of the known type are unable to function in the absence of mains power and moreover are subject to safety regulations which increase considerably the production costs thereof.

The object of the present invention is to overcome the drawbacks mentioned above and in particular to be able to use the device in any fixed or mobile environemnt or in the open.

A further object is to provide a device which is safer and not subject to certification or safety regulations. Said objects as well as others are all achieved by the device for the atomization of liquid substances, which is characterized by the contents of the accompanying claims.

The characteristic features and advantages will emerge more clearly from the following description of a preferred embodiment illustrated, purely by way of a nonlimiting example, in the accompanying illustrative plate, in which:

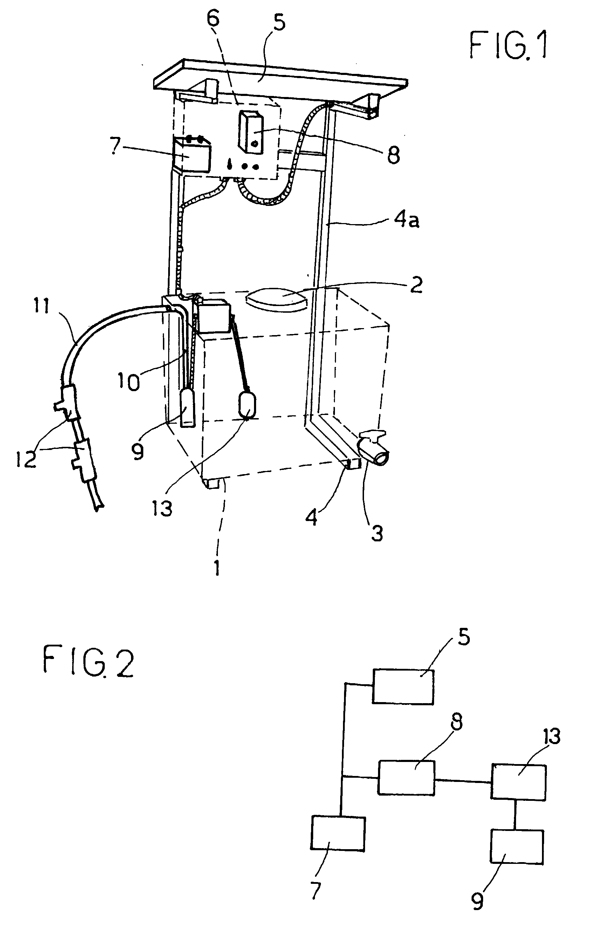

- Figure 1 shows a perspective view of the device;
- Figure 2 is a block diagram illustrating interconnection of the main components of the device. With reference to the Figure, 1 denotes a tank into which a liquid is introduced through an opening closed by a lid 2.

At the bottom of the tank there is provided a tap 3 for discharging liquid if required.

The tank rests on a frame 4 which has two uprights 4a which support a solar panel 5 whose direction can be adjusted and which supplies power to an electric console 6 provided with a battery 7 which powers a timer 8 which, in turn, with a certain frequency, powers a pump of the submergible type 9 contained in the tank 1 from which it draws the liquid.

The submergible pump 9 is connected, by means of a pipe 10, to a line 11 along which there are arranged atomization nozzles 12, the number of which will be variable and depend on the size of the room or external environment being treated.

The electric circuit inside the electric console is structured so that, during the day, the pump is powered directly by the solar panel, while at nighttime it is powered via the battery which is recharged during the day again by the solar panel.

Adavantageously the electric console may be provided with devices indicating the charge level of the battery as well as devices indicating operation of the solar panel. The tank is provided internally with a float 13 which is electrically connected to the electric console so as to define a minimum level beyond which operation of the device is prevented.

The principal advantage of invention described above is that it is able to operate in any location and at any time, and is extremely simple and safe since it operates with low voltage.

According to a possible variation the tank could be divided into two or more compartments so as to contain different liquids, in which case two or more submergible pumps which can be activated independently of one another must be provided.

## Claims

1. Device for the atomization of liquid substances of the type comprising a tank containing a liquid and a plurality of atomization nozzles, characterized in that it comprises in combination: a pump of the submergible type (9) contained inside the tank and connected to the pluralty of atomization nozzles; an electric battery (7) powering the pump; a solar panel (5) designed to power directly the submergible pump (9) or to charge the electric battery (7).

2. Device according to Claim 1, characterized in that it comprises a float (13) located inside the tank so as to determine a minimum level below which operation of the device is prevented by means of interruption of the power supply of the pump.

3. Device according to Claim 1, characterized in that it comprises a timer (8) designed to power cyclically the pump (9).

4. Device according to Claim 1, characterized in that it comprises a tank divided up into several compartments, each of which contains a pump of the submergible type which can be activated simultaneously with or independently of the other ones.
